# EUROPEAN PATENT APPLICATION

(11) **EP 4 778 522 A1**
(43) Date of publication of application: **22.07.2026**
(21) Application number: 24864622.6
(22) Date of filing: 10.09.2024
(51) Int. Cl.: A61K 31/519, C07D 519/00, A61P 37/02, A61P 17/00, A61P 17/14, A61P 29/00

(54) **USE OF PYRROLOPYRIMIDINE COMPOUND AND COMPOSITION THEREOF**

(30) Priority: 12.09.2023 CN 202311174088
(71) Applicant: Guangzhou Joyo Pharmatech Co., Ltd., Guangzhou, Guangdong 510663 (CN)
(72) Inventor: LI, Yongguo, Guangzhou, Guangdong 510663 (CN); CHEN, Xiaoning, Guangzhou, Guangdong 510663 (CN); SONG, Haoliang, Guangzhou, Guangdong 510663 (CN); LI, Hongjiao, Guangzhou, Guangdong 510663 (CN); LI, Zongrui, Guangzhou, Guangdong 510663 (CN); MA, Hongyan, Guangzhou, Guangdong 510663 (CN); GE, Zhen, Guangzhou, Guangdong 510663 (CN); HUANG, Huipiao, Guangzhou, Guangdong 510663 (CN)
(74) Representative: Michalski Hüttermann & Partner mbB
(86) International application number: PCT/CN2024/118038
(87) International publication number: WO 2025/055906

(57) **Abstract**

The present invention relates to the use of a pyrrolopyrimidine compound and a composition thereof, particularly the use of a compound represented by formula I, a pharmaceutically acceptable salt or a pharmaceutical composition thereof in the manufacture of a drug for treating and/or preventing autoimmune skin diseases. The drug can effectively treat and/or prevent autoimmune skin diseases, especially atopic dermatitis, vitiligo and alopecia areata.

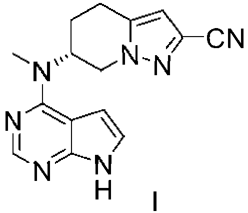

## Description

The present application claims the priority of Chinese patent application 2023111740884 filed on September 12, 2023. The contents of the Chinese patent application are incorporated herein by reference in their entireties.

### TECHNICAL FIELD

The present disclosure relates to use of a pyrrolopyrimidine compound in the manufacture of a drug and a composition thereof.

### BACKGROUND

Autoimmune skin diseases are a class of skin diseases with skin damages due to immune imbalance caused by immune response against autoantigens elicited by an immune system, including vitiligo, psoriasis, atopic dermatitis, alopecia areata and the like. The traditional treatment schemes for autoimmune skin diseases mainly comprise glucocorticoids, traditional small molecule immunosuppressants and the like, but their clinical curative effects are not satisfactory. Besides, the unavoidable toxic and side effects brought by long-term use of such drugs become a huge problem. Therefore, it is urgent to develop a new drug with a good curative effect, high safety and less adverse reactions aiming at the existing targets and new targets, so as to solve the unsatisfied clinical requirements and improve the life quality of patients.

Atopic dermatitis (AD) is a chronic, recurrent and inflammatory skin disease whose onset is closely related with genetic and environmental factors and the like. Topical glucocorticoids (TCS) are currently the first line treatment of AD, but the long-term large-area use of TCS may lead to adverse reactions of the skin and system. Topical calcineurin inhibitors (TCI) are important anti-inflammatory drugs for the treatment of AD, which can be used for controlling inflammation and itching symptoms at the face, neck and fold parts, or for actively maintaining treatment and reducing recurrence. The main adverse reactions include local burning sensation and irritation. For AD patients with moderate-severe symptoms or poor local curative effect, they often need systemic treatment, mainly including oral administration of antihistamines, small molecule immunosuppressants, biological preparations and the like. The antihistamines may affect sleep quality and learning and cognitive abilities, and thus are not recommended for long-term use; adverse reactions of the traditional small molecule inhibitors such as methotrexate and cyclosporine need to be monitored closely, and thus their uses are limited; and although the antibodies are more accurately targeted, for example, dupilumab is a fully human monoclonal antibody for interleukin (IL)-4 receptors and has a good curative effect for adult and child moderate-severe patients, and the proportion of its adverse reaction conjunctivitis can reach 10% or more.

Vitiligo is a common acquired depigmentation disease, which has main clinical manifestations such as hypopigmented and/or depigmented patches. Since vitiligo has a complex etiology and its pathogenesis remains unclear, it is widely believed that the killing of CD8+ T lymphocytes on melanocytes is a direct reason for causing vitiligo at present. The treatment of vitiligo is intended to control the progress of vitiligo and promote the color recovery of white patches. The current treatment methods are various and mainly include systemic treatment, topical drug treatment, physical treatment, surgical treatment and the like. Vitiligo is stubborn, easy to recur and difficult to treat, and often causes great psychological influence on patients.

Alopecia areata is a type of T cell-mediated hair follicle specific inflammatory and non-cicatricial alopecia, belonging to the relatively common autoimmune related skin diseases. It is usually clinically manifested as circular or oval single or multiple patchy alopecia occurring suddenly or in a short time, with a clear boundary and an unequal size, and is mainly seen in hairs and can also affect other parts of the body. The appearance of the skin in the alopecia areata is basically normal, no obvious inflammatory reaction, atrophy and cicatrices exist, but the appearance and the life quality of patients are obviously negatively affected. Some patients can also be complicated by autoimmune or inflammatory diseases such as lupus erythematosus, atopic dermatitis and vitiligo. The alopecia areata is not completely clear in pathogenesis, which is mainly caused by the combined action of genetic factors and environmental factors, and currently thought to be associated with hair follicle immune privilege damage resulting from an imbalance of multiple immune genes, immune cells and immune molecules. The treatment of alopecia areata is intended to control disease progression, promote hair regrowth, prevent or reduce recurrence, and improve the life quality of patients. Most patients with mild alopecia areata can be self-healed, but about half experience recurrent episodes, and the disease can follow a chronic course lasting for years or decades; and patients with moderate and severe alopecia areata have serious conditions, with hair loss potentially affecting the entire scalp or even body hair, and their treatment still faces challenges.

### CONTENT OF THE PRESENT INVENTION

The present disclosure relates to use of a compound shown in Formula I, a pharmaceutically acceptable salt thereof or a pharmaceutical composition in the manufacture of a drug for treating and/or preventing an autoimmune skin disease. The present disclosure can effectively treat and/or prevent the autoimmune skin disease, especially atopic dermatitis, vitiligo and alopecia areata.

The present disclosure provides use of a compound shown in Formula I, a pharmaceutically acceptable salt thereof or a pharmaceutical composition in the manufacture of a drug for treating and/or preventing an autoimmune skin disease, wherein the autoimmune skin disease is one or more of atopic dermatitis, vitiligo and alopecia areata, the pharmaceutical composition comprises the compound shown in Formula I or the pharmaceutically acceptable salt thereof, and at least one pharmaceutically acceptable carrier,

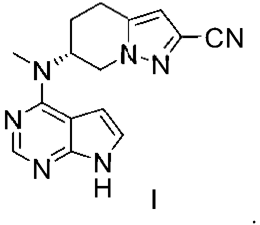

In a certain embodiment, the pharmaceutical composition consists of the compound shown in Formula I or the pharmaceutically acceptable salt thereof and at least one pharmaceutically acceptable carrier.

In a certain embodiment, the pharmaceutical composition is an oral preparation or a topical preparation.

In a certain embodiment, the pharmaceutical composition is a capsule, a tablet, a granule, a powder, a lotion, a tincture, a liniment, a paint, an ointment, a gel, a paste, a transdermal patch or an aerosol, preferably an ointment or a gel.

In a certain embodiment, the atopic dermatitis has one or more of the following characteristics:
(1) hyperkeratosis of follicular infundibulum;
(2) thickening of epidermis;
(3) inflammatory cell infiltration; and
(4) enlargement of follicular orifice.

In a certain embodiment, the vitiligo is preferably segmental vitiligo, non-segmental vitiligo (vitiligo vulgaris), mixed vitiligo (coexistence of segmental vitiligo and non-segmental vitiligo) or indeterminate vitiligo (original localized type).

In a certain embodiment, the alopecia areata is preferably of a patch type, a net type, an ophiasis type, a central type, a reverse-ophiasis type, a diffuse type, alopecia totalis or alopecia universalis.

In a certain embodiment, the pharmaceutically acceptable salt can be fumarate, adipate, phosphate, tartrate, maleate, hydrochloride, citrate, sulfate, methanesulfonate, benzenesulfonate or p-toluenesulfonate, preferably the sulfate.

In a certain embodiment, the compound shown in Formula I, the pharmaceutically acceptable salt thereof or the pharmaceutical composition is evenly applied to the surface of skin at a use frequency according to the adaptation of the skin.

In a certain embodiment, calculated as the compound shown in Formula I, the compound shown in Formula I, the pharmaceutically acceptable salt thereof or the pharmaceutical composition is administered at a total dose of 0.1-200 mg every day, such as 0.2 mg, 0.5 mg, 1 mg, 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg.

In a certain embodiment, calculated as the compound shown in Formula I, each minimum drug content specification contains 0.1-200 mg of the compound shown in Formula I, such as 0.2 mg, 0.5 mg, 1 mg, 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg; preferably 1 mg, 5 mg, 10 mg, 15 mg, 30 mg, 45 mg, 60 mg, 75 mg, 90 mg or 120 mg; more preferably 5 mg, 10 mg, 15 mg, 30 mg, 45 mg, 60 mg, 75 mg or 90 mg; or calculated as the compound shown in Formula I, each minimum drug content specification contains 0.5 wt%-5.0 wt% of the compound shown in Formula I, for example 0.5 wt%-2 wt%.

In a certain embodiment, when the compound shown in Formula I, the pharmaceutically acceptable salt thereof or the pharmaceutical composition is applied topically, the dose calculated as the compound shown in Formula I is 0.001-1 mg/cm², preferably 0.005-0.5 mg/cm², more preferably 0.01-0.2 mg/cm², such as 0.01 mg/cm², 0.03 mg/cm², 0.05 mg/cm², 0.07 mg/cm², 0.08 mg/cm², 0.1 mg/cm², 0.15 mg/cm² or 0.2 mg/cm².

In a certain embodiment, when the compound shown in Formula I, the pharmaceutically acceptable salt thereof or the pharmaceutical composition is orally taken, the total daily dose calculated as the compound shown in Formula I is 1-200 mg, preferably 5-200 mg, such as 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg.

In a certain embodiment, when the autoimmune skin disease is atopic dermatitis, the dose calculated as the compound shown in Formula I is 0.005-0.5 mg/cm², preferably 0.01-0.2 mg/cm², such as 0.01 mg/cm², 0.03 mg/cm², 0.05 mg/cm², 0.07 mg/cm², 0.08 mg/cm², 0.1 mg/cm², 0.15 mg/cm² or 0.2 mg/cm².

In a certain embodiment, when the autoimmune skin disease is vitiligo, the dose calculated as the compound shown in Formula I is 0.005-0.5 mg/cm², preferably 0.01-0.2 mg/cm², such as 0.01 mg/cm², 0.03 mg/cm², 0.05 mg/cm², 0.07 mg/cm², 0.08 mg/cm², 0.1 mg/cm², 0.15 mg/cm² or 0.2 mg/cm².

In a certain embodiment, when the autoimmune skin disease is alopecia areata, the dose calculated as the compound shown in Formula I is 1-200 mg, preferably 5-200 mg, such as 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg.

In a certain embodiment, the administration frequency of the compound shown in Formula I, the pharmaceutically acceptable salt thereof or the pharmaceutical composition is once every two days, once every day, twice every day or three times every day, preferably once every day or twice every day.

In a certain embodiment, calculated as the compound shown in Formula I, the compound shown in Formula I or the pharmaceutically acceptable salt thereof are administered at 5-90 mg per dose (such as 5 mg, 10 mg, 15 mg, 30 mg, 45 mg, 60 mg, 75 mg, or 90 mg).

In a certain embodiment, the administration mode of the compound shown in Formula I, the pharmaceutically acceptable salt thereof or the pharmaceutical composition is oral administration, parenteral administration or transdermal administration, preferably oral administration or transdermal administration.

In a certain embodiment, when the autoimmune skin disease is atopic dermatitis or vitiligo, the administration mode of the compound shown in Formula I, the pharmaceutically acceptable salt thereof or the pharmaceutical composition is transdermal administration.

In a certain embodiment, when the autoimmune skin disease is alopecia areata, the administration mode of the compound shown in Formula I, the pharmaceutically acceptable salt thereof or the pharmaceutical composition is oral administration, parenteral administration or transdermal administration, preferably oral administration.

In a certain embodiment, when the administration mode of the compound shown in Formula I, the pharmaceutically acceptable salt thereof or the pharmaceutical composition is transdermal administration, the dosage form of the transdermal administration is the gel; preferably, calculated as the compound shown in Formula I, the gel contains 0.5 wt%, 1.0 wt% or 2.0 wt% of the compound shown in Formula I.

In some embodiments, when the autoimmune skin disease is atopic dermatitis, the compound shown in Formula I, the pharmaceutically acceptable salt thereof or the pharmaceutical composition is used by transdermal administration twice every day at the dose of 0.005-0.1 mg/cm² each time calculated as the compound shown in Formula I;
In some embodiments, when the autoimmune skin disease is vitiligo, the compound shown in Formula I, the pharmaceutically acceptable salt thereof or the pharmaceutical composition is used by transdermal administration twice every day at the dose of 0.005-0.1 mg/cm² each time calculated as the compound shown in Formula I.

In some embodiments, when the autoimmune skin disease is the alopecia areata, the compound shown in Formula I, the pharmaceutically acceptable salt thereof or the pharmaceutical composition is used by oral administration twice every day at the dose of 5-90 mg each time calculated as the compound shown in Formula I.

In some embodiments, when the pharmaceutical composition is the oral preparation, the dose of the compound shown in Formula I and the pharmaceutically acceptable salt thereof in the pharmaceutical composition is 5-200 mg, such as 5 mg, 10 mg, 15 mg, 30 mg, 45 mg, 60 mg, 75 mg, 90 mg, 100 mg, 120 mg, 115 mg, 130 mg, 145 mg, 160 mg, 175 mg or 190 mg calculated as the compound shown in Formula I.

In some embodiments, when the pharmaceutical composition is the gel, the pharmaceutical composition is a pharmaceutical composition A comprising the following components:
an active ingredient, wherein the active ingredient is the compound shown in Formula I or the pharmaceutically acceptable salt thereof; and the active ingredient accounts for 0.5 wt%-2 wt% of the pharmaceutical composition calculated as the compound shown in Formula I;
5.0 wt%-7.0 wt% of Sepineo P 600;
20.0 wt% of diethylene glycol monoethyl ether;
15.0 wt% of glycerol; and
propylene glycol q.s. to 100% of the total composition.

In a certain embodiment, the pharmaceutical composition A contains an active ingredient, wherein the active ingredient is the sulfate of the compound shown in Formula I and the active ingredient accounts for 2 wt% of the pharmaceutical composition calculated as the compound shown in Formula I;
7.0 wt% of Sepineo P 600;
20.0 wt% of diethylene glycol monoethyl ether;
15.0 wt% of glycerol; and propylene glycol q.s. to 100% of the total composition.

In a certain embodiment, the pharmaceutical composition A contains an active ingredient, wherein the active ingredient is the sulfate of the compound shown in Formula I and the active ingredient accounts for 1 wt% of the pharmaceutical composition calculated as the compound of Formula I;
6.0 wt% of Sepineo P 600;
20.0 wt% of diethylene glycol monoethyl ether;
15.0 wt% of glycerol; and propylene glycol q.s. to 100% of the total composition.

In a certain embodiment, the pharmaceutical composition A contains an active ingredient, wherein the active ingredient is the sulfate of the compound shown in Formula I and the active ingredient accounts for 0.5 wt% of the pharmaceutical composition calculated as the compound shown in Formula I;
5.0 wt% of Sepineo P 600;
20.0 wt% of diethylene glycol monoethyl ether;
15.0 wt% of glycerol; and propylene glycol q.s. to 100% of the total composition.

The present disclosure further provides a pharmaceutical composition as shown in the pharmaceutical composition A in the use as described above.

The present disclosure further provides a method for treating or preventing an autoimmune skin disease, comprising administering to a subject in a need thereof (a therapeutically effective amount of) the compound shown in Formula I, the pharmaceutically acceptable salt thereof or the pharmaceutical composition. The autoimmune skin disease is one or more of atopic dermatitis, vitiligo and alopecia areata.

In a certain embodiment, the autoimmune skin disease is as described above.

In a certain embodiment, the administration dose of the compound shown in Formula I, the pharmaceutically acceptable salt thereof or the pharmaceutical composition is as described above.

In a certain embodiment, the administration mode of the compound shown in Formula I, the pharmaceutically acceptable salt thereof or the pharmaceutical composition is as described above.

In a certain embodiment, the administration frequency of the compound shown in Formula I, the pharmaceutically acceptable salt thereof or the pharmaceutical composition is as described above.

In a certain embodiment, the treatment can ameliorate/reduce the hyperkeratosis of the follicular infundibulum (compared to model control), for example, the pathological index score of a keratinous substance of the follicular infundibulum is reduced by 35%-68%.

In a certain embodiment, the treatment can ameliorate/reduce the thickening of the epidermis and inflammatory cell infiltration (compared to model control), for example, the pathological index score of the thickening of the epidermis and the inflammatory cell infiltration is reduced by 38%-52%.

In a certain embodiment, the treatment can ameliorate/reduce the enlargement of the follicular orifice (compared to model control), for example, the pathological index score of the enlargement of the follicular orifice is reduced by 25%-55%.

The pathological index scoring criteria are as follows:

| Pathological examination index | Scoring criteria | Score |
|---|---|---|
| I. Dense keratinous substance of follicular infundibulu m; | Slightly dense keratinous substance | 1 |
| | Accumulation of small amount of dense keratinous substance | 2 |
| | Denser keratinous substance extending to sebaceous glands | 3 |
| | Wide keratinous substance extending to sebaceous glands | 4 |
| II. Thickening of superficial squamous epithelium, inflammator y cell infiltration, congestion and redness | Slight thickening of superficial squamous epithelium | 1 |
| | Obvious thickening of superficial squamous epithelium | 2 |
| | Obvious thickening of superficial squamous epithelium and inflammatory cell infiltration | 3 |
| | Obvious thickening of superficial squamous epithelium, inflammatory cell infiltration, and obvious congestion and redness | 4 |
| III. Enlargement | Slight enlargement of follicular orifice (1-2 times greater than normal group) | 1 |
| | Enlargement of follicular orifice (2-3 times greater than normal group) | 2 |
| | Enlargement of follicular orifice (3-4 times greater than normal group) | 3 |
| of follicular orifice | Obvious enlargement of follicular orifice (4 times or greater than normal group) | 4 |
| Total maximum score | | 12 |

In a certain embodiment, the treatment can ameliorate/reduce the hair depigmentation area of vitiligo (compared to model control), for example, the hair depigmentation area score is reduced by 50%-60%.

The hair depigmentation scoring criteria are as follows:

| Score | Proportion of hair depigmentation area |
|---|---|
| 0 | No hair depigmentation |
| 1 | 0-10% of hair depigmentation area |
| 2 | 11%-25% of hair depigmentation area |
| 3 | 26%-50% of hair depigmentation area |
| 4 | 51%-75% of hair depigmentation area |
| 5 | 76%-100% of hair depigmentation area |

In a certain embodiment, the treatment can increase the number of the melanin-containing hair follicles and increase the proportion of the melanin-containing hair follicles (compared to model control), for example, the number of the melanin-containing hair follicles is increased by 300%-350%, and the proportion of the melanin-containing hair follicles is increased by 110%-120%.

In a certain embodiment, the treatment can increase the number of the hair follicles in the alopecia areata part (compared to model control), for example, the number of the hair follicles in the alopecia areata part is increased by 400%-1000%.

The present disclosure further provides a substance X for treating an autoimmune skin disease. The substance X is the compound shown in Formula I, the pharmaceutically acceptable salt thereof or the pharmaceutical composition, wherein the pharmaceutical composition comprises the compound shown in Formula I or the pharmaceutically acceptable salt thereof, and at least one pharmaceutically acceptable carrier, and the autoimmune skin disease is one or more of atopic dermatitis, vitiligo and alopecia areata.

In a certain embodiment, the autoimmune skin disease is as described above.

In a certain embodiment, the pharmaceutically acceptable salt shown in Formula I is fumarate, adipate, phosphate, tartrate, maleate, hydrochloride, citrate, sulfate, methanesulfonate, benzenesulfonate or p-toluenesulfonate, preferably the sulfate.

In a certain embodiment, the pharmaceutical composition is as described above.

In a certain embodiment, the administration dose of the compound shown in Formula I, the pharmaceutically acceptable salt thereof or the pharmaceutical composition is as described above.

In a certain embodiment, the administration mode of the compound shown in Formula I, the pharmaceutically acceptable salt thereof or the pharmaceutical composition is as described above.

In a certain embodiment, the administration frequency of the compound shown in Formula I, the pharmaceutically acceptable salt thereof or the pharmaceutical composition is as described above.

In a certain embodiment, the treatment is as described above.

### Definition and description

Unless otherwise indicated, the following terms and phrases used herein are intended to have the following meanings. Unless otherwise specifically defined, a particular term or phrase should not be considered as indefinite or unclear, but rather construed according to ordinary meaning. When a trade name appears herein, it is intended to refer to its corresponding commercial product or its active ingredient.

The vitiligo comprises segmental vitiligo, vitiligo vulgaris, mixed vitiligo or indeterminate vitiligo.

The segmental vitiligo refers to unilateral asymmetric vitiligo distributed along a certain cutaneous nerve segment (completely or partially matched with a skin segment), and a few bilaterally distributed in multiple segments;
the non-segmental vitiligo (vitiligo vulgaris) comprises sporadic vitiligo, generalized vitiligo, facial cervical vitiligo, acral vitiligo and mucosal vitiligo; the sporadic vitiligo means that the number of white patches is ≥ 2 and the area is grade 1-3; the generalized vitiligo means that the area of the white patch is grade 4 (> 50%); and the facial cervical vitiligo, the acral vitiligo and the mucosal vitiligo are all developed into the generalized vitiligo;
the mixed vitiligo: the segmental vitiligo coexists with the non-segmental vitiligo; and
the indeterminate vitiligo (original localized type): a single patch skin is injured, the area is grade 1, and the vitiligo cannot be determined to be segmental or non-segmental.

The alopecia areata comprises a plurality of types: patch type, net type, ophiasis type, central type or reverse-ophiasis type, diffuse type, alopecia totalis or alopecia universalis.

The patch type: a hair loss patch may be single or multiple, round or oval, has clear boundary, and is easy to recover for one a small area;
the net type: the hair loss patches are many and dense and in a net shape;
the ophiasis type, i.e. the band type: it mainly occurs at a hairline part, and the therapeutic response is often poor;
the central type or the reverse-ophiasis type;
the diffuse type: it diffusely affects the whole scalp, usually has an acute episode, and generally does not form alopecia totalis, new hairs usually grow before old hairs completely fall off, the patchy alopecia may be found by careful examination, and an acutely-affected patient may recover easily;
the alopecia totalis: all hairs on the head fall off; and
the alopecia universalis: all hairs throughout the body fall off.

The terms "compound shown in Formula I" or "pharmaceutically acceptable salt of compound shown in Formula I" is defined to include all forms of the compound of Formula (I) or the pharmaceutically acceptable salt of the compound shown in Formula I, including its crystalline and amorphous forms, polymorphs, solvates and hydrates.

The term "pharmaceutically acceptable salt thereof" includes, but is not limited to fumarate, adipate, phosphate, tartrate, maleate, hydrochloride, citrate, sulfate, methanesulfonate, benzenesulfonate or p-toluenesulfonate, preferably the sulfate.

The term "treatment" refers to a therapeutic therapy. When referring to specific disorders, the treatment refers to: (1) relieving one or more biological manifestations of a disease or disorder, (2) interfering with (a) one or more points in a biological cascade that causes or contributes to a disorder or (b) one or more biological manifestations of a disorder, (3) ameliorating one or more symptoms, effects, or side effects associated with a disorder, or one or more symptoms, effects, or side effects associated with a disorder or treatment thereof, or (4) slowing the progression of a disorder or one or more biological manifestations of the disorder.

The term "prevention" refers to a reduction in the risk of acquiring or developing a disease or disorder.

The term "therapeutically effective amount" refers to an amount of a compound that, when administered to a subject, is sufficient to effectively treat a disease or disorder described herein. The "therapeutically effective amount" will vary depending on the compound, the disorder and its severity, and the age of a patient to be treated, but may be adjusted as desired by those skilled in the art.

The term "subject" refers to any animal, preferably a mammal, most preferably a human, that is to be or has received administration of the compound according to embodiments of the present disclosure. The term "mammal" comprises any mammals. Examples of the mammals include but are not limited to cows, horses, sheep, pigs, cats, dogs, mice, rats, rabbits, guinea pigs, monkeys, humans and the like, most preferably a human.

Without prejudice to common knowledge in the art, the above-mentioned preferred conditions can be combined arbitrarily to obtain preferred embodiments of the present disclosure.

The reagents and raw materials used in the present disclosure are all commercially available.

The present disclosure has the following positive effects: the present disclosure relates to use of the compound shown in Formula I, the pharmaceutically acceptable salt thereof or the pharmaceutical composition in the preparation of the drug for treating and/or preventing the autoimmune skin disease. The present disclosure can effectively treat and/or prevent the autoimmune skin disease, especially atopic dermatitis, vitiligo and alopecia areata, and has a better therapeutic effect and safety and fewer side effects or adverse reactions than the existing treatment schemes or drugs.

### BRIEF DESCRIPTION OF THE DRAWINGS

FIG. 1 shows hair growth at alopecic areas of mice in each experimental group in Example 3; and
FIG. 2 shows HE staining and mean hair follicle number of skin tissues at alopecic areas of mice in each experimental group in Example 3, wherein A is the HE staining result of the skin tissues at the alopecic areas; and B is the mean hair follicle number of the skin tissues at the alopecic areas, expressed as Mean ± SEM, * P < 0.05, ** P < 0.01 and **** P < 0.001.

### DETAILED DESCRIPTION

Next, the present disclosure will be further described with reference to embodiments, but not limited to the scope of the described embodiments. The experimental methods in the following embodiments without specified specific conditions should be selected according to conventional methods and conditions, or according to instructions of products.

### Example 1 Drug efficacy experiment of fluorescein isothiocyanate (FITC)-induced mouse atopic dermatitis model

### 1. Objective

To evaluate the therapeutic effect of test compound I on an FITC-induced mouse atopic dermatitis model

### 2. Method

1) Animals: female Balb/c mice, specific pathogen-free (SPF), aged 6-7 weeks at grouping, with 10 mice per group. The animals were provided by Shanghai Jihui Laboratory Animal Care Co., Ltd.
2) Reagents, positive drugs and test drugs

### A. Reagents

The sources of the reagents are shown in Table 1.

**Table 1 Sources of reagents**

| Name | Supplier |
|---|---|
| FITC (fluorescein isothiocyanate) | Sigma-Aldrich |
| Dibutyl phthalate | Aladdin |
| Acetone | Sinopharm |
| Isoflurane | Hebei Jindafu Pharmaceutical Co., Ltd |
| Zoletil | Virbac |
| Xylazine | Tokyo |
| Hematoxylin dye liquor | Wuxi Jiangyuan Industrial Technology and Trade Corporation |
| Eosin dye liquor | Wuxi Jiangyuan Industrial Technology and Trade Corporation |

B. Positive drug: clobetasol propionate ointment, which was provided by Shanghai New Asiatic Pharmaceutical.

C. Test drug: compound I, a salt of the compound I and a hydrate of the salt, which were provided by Guangzhou JOYO Pharmatech Co., Ltd. The compound I was also prepared according to Example 1 in WO 2020244614 A1; and the salt of the compound I and the hydrate of the salt were also prepared by the method disclosed in WO 2022117111 A1.

Compound I gel (provided by Guangzhou JOYO Pharmatech Co., Ltd.): the Compound I gel with the mass fractions of 0.5%, 1.0% and 2.0% was prepared from Compound I sulfate hydrate, respectively. By taking the Compound I gel with the mass fraction of 2.0% as an example, the mass fraction of each component is shown in Table 2 below.

**Table 2**

| Proportion of raw and auxiliary materials | (w/w)% |
|---|---|
| Compound I sulfate hydrate | 2.0^{a} |
| Sepineo P 600 | 7.0 |
| Diethylene glycol monoethyl ether | 20.0 |
| Glycerol | 15.0 |
| Propylene glycol | Making up to 100% |
| Total | 100.0 |

| | |
|---|---|
| a. The mass fraction of Compound I in Compound I sulfate hydrate in a gel composition | |

The preparation method of the Compound I with the mass fractions of 0.5% and 1.0% in the gel was different from that of the Compound I with the mass fraction of 2.0% in the gel in that: (1) the mass fractions of Sepineo P600 were different, which were 5.0% and 6.0% respectively; and (2) the mass fractions of the Compound I sulfate hydrate were different, and the mass fractions of the Compound I in the gel composition were 0.5% and 1.0% respectively; and propylene glycol made up to 100% for the mass fraction of the changed parts of the total amounts of the two components.

### 3) Modeling method

FITC induction method: experimental animals were subjected to examination in an adaptation period and weight screening after examined to be qualified. The animals were randomly divided into 6 experimental groups according to the weight, including a normal group, a model group, a clobetasol propionate ointment group, a 0.5% Compound I group, a 1% Compound I group and a 2% Compound I group, with 10 mice in each group. On day 0 and day 1, after all the experimental animals were administered for 2 hours, the animals were anesthetized with zoletil (25 mg/kg) and xylazine (5 mg/kg), and then subjected to sensitization operation at the abdomen (a sensitized part). The abdominal part of the experimental animal at about 3×4 cm was depilated before sensitization, and then 0.5% FITC (dissolved in dibutyl phthalate/acetone solution) was applied to the alopecic areas of the abdomens of the experimental animals in model group for sensitization. An equal volume of dibutyl phthalate/acetone was given to the alopecic areas of the abdomens of the experimental animals in the normal-blank gel group.

On day 5 and day 12, all the experimental animals were anesthetized with 1-4% isoflurane (all subsequent measurements on ear thickness were performed under anesthesia with isoflurane), and the thickness of the right-side ear margins of the experimental animals was measured as a baseline. On day 5 and day 12, 0.5% FITC was applied to both the inner and outer sides of the right ears of the experimental animals in model group for challenge treatment. An equal volume of dibutyl phthalate/acetone solution was given to the experimental animals in the normal-blank gel group.

On day 6 and day 13, 24 hours after each FITC challenge, the thickness of the right-side ear margins of all the experimental animals was measured. Ear swelling response was determined by the change in the ear thickness before and after FITC challenge measured with a digital display thickness gauge, the measurement positions were basically kept constant, and the mean change in the ear thickness (ΔT±S.E.M.) was recorded for each group.

### 4) Grouping and administration

The grouping and administration regimens are shown in Table 3 below.

**Table 3 Grouping and administration regimen**

| Group | Test substance | Number of animals | Administration route | Concentration | Dose | Administration regimen |
|---|---|---|---|---|---|---|
| 1 | Normal-blank gel | 10 | Topical | N/A | 10 mg/dose | Applied twice a day from Day 1 |
| 2 | Model-blank gel | 10 | Topical | N/A | 10 mg/dose | Applied twice a day from Day 1 |
| 3 | Model-clobetasol propionate ointment | 10 | Topical | 0.2% | 10 mg/dose | Applied twice a day from Day 1 |
| 4 | Model-0.5% compound I | 10 | Topical | 0.5% | 20 mg/dose | Applied twice a day from Day 1 |
| 5 | Model-1% compound I | 10 | Topical | 1% | 20 mg/dose | Applied twice a day from Day 1 |
| 6 | Model-2% compound I | 10 | Topical | 2% | 20 mg/dose | Applied twice a day from Day 1 |

### 5) Endpoint sample collection

On day 13, all the experimental animals were euthanized by CO₂ and cervical dislocation, and then the right ears were collected and fixed in 10% formalin for histopathological analysis.

### 6) Histopathological scoring

After the ear tissues were embedded with paraffin, each ear tissue was sliced into 3-5 µm sections. After HE staining, the sections were subjected to routine histopathological evaluation and ear tissue thickness measurement. The histopathological scoring criteria are as shown in Table 4 below.

**Table 4 Histopathological scoring criteria**

| Pathological examination index | Scoring criteria | Score |
|---|---|---|
| I. Dense keratinous substance of follicular infundibulum; | Slightly dense keratinous substance | 1 |
| | Accumulation of small amounts of dense keratinous substance | 2 |
| | Denser keratinous substance extending to sebaceous glands | 3 |
| | Wide keratinous substance extending to sebaceous glands | 4 |
| II. Thickening of superficial squamous epithelium, inflammatory cell infiltration, congestion and redness | Slight thickening of superficial squamous epithelium | 1 |
| | Obvious thickening of superficial squamous epithelium | 2 |
| | Obvious thickening of superficial squamous epithelium and inflammatory cell infiltration | 3 |
| | Obvious thickening of superficial squamous epithelium, inflammatory cell infiltration, and obvious congestion and redness | 4 |
| III. Enlargement of follicular orifice | Slight enlargement of follicular orifice (1-2 times greater than normal group) | 1 |
| | Enlargement of follicular orifice (2-3 times greater than normal group) | 2 |
| | Enlargement of follicular orifice (3-4 times greater than normal group) | 3 |
| | Obvious enlargement of follicular orifice (4 times or greater than normal group) | 4 |
| Total maximum score | | 12 |

### 3. Results

### 1) Change in ear thickness of experimental animals

**Table 5 Increased thickness of right ears of experimental animals**

| Groups | | Ear thickness (mm) | | | | ΔEar thickness (mm) | | |
|---|---|---|---|---|---|---|---|---|
| | | Day 5 | Day 6 | Day 12 | Day 13 | Day 6-Day 5 | Day 13-Day 5 | Inhibitory rate% (Day 13) |
| Gl-normal group | Mean | 0.15 | 0.16 | 0.15 | 0.16 | 0.0090 | 0.0080 | / |
| | SEM | 0.00 | 0.00 | 0.00 | 0.00 | 0.0010 | 0.0020 | / |
| G2-model group | Mean | 0.17 | 0.20 | 0.18 | 0.26 | 0.0290 | 0.0980 | / |
| | SEM | 0.00 | 0.00 | 0.00 | 0.00 | 0.0035 | 0.0025 | / |
| G3-clobetasol propionate ointment group | Mean | 0.14 | 0.14 | 0.16 | 0.16 | 0.0020 | 0.0210 | 78.57%**** |
| | SEM | 0.00 | 0.00 | 0.00 | 0.00 | 0.0013 | 0.0043 | / |
| G4-0.5% compound 1 group | Mean | 0.17 | 0.19 | 0.18 | 0.19 | 0.0250 | 0.0170 | 82.65%**** |
| | SEM | 0.00 | 0.00 | 0.00 | 0.00 | 0.0017 | 0.0033 | / |
| G5-1% compound I group | Mean | 0.17 | 0.20 | 0.18 | 0.19 | 0.0280 | 0.0250 | 74.49%**** |
| | SEM | 0.00 | 0.00 | 0.00 | 0.00 | 0.0020 | 0.0043 | / |
| G6-2% compound I group | Mean | 0.17 | 0.20 | 0.18 | 0.20 | 0.0260 | 0.0250 | 74.49%**** |
| | SEM | 0.00 | 0.00 | 0.00 | 0.00 | 0.0034 | 0.0017 | / |

Results: as shown in Table 5, after the first FITC challenge, the thickness of the right ears of the animals in model group was increased to a certain extent on day 6, but the ears showed no change in appearance. After the second FITC challenge, the right ears of the animals in G2 model group showed obvious swelling and thickening on day 13, and the Δ ear thickness value was obviously increased compared with that in G1 normal group (0.0980 ± 0.0025 mm vs. 0.0080 ± 0.0020 mm, and P < 0.0001). In positive drug clobetasol propionate ointment group (G3), the swelling of the right ears of the animals was obviously relieved, the Δ ear thickness value was obviously reduced compared with that in G2 model group (0.0210 ± 0.0043 mm vs. 0.0980 ± 0.0025 mm, P < 0.0001), and the inhibitory rate was 78.57%. The test compound I topical preparation had a good therapeutic effect on swelling and thickening symptoms of the right ears of the animals at concentrations of 0.5%, 1% and 2%, the Δ ear thickness values were 0.0170 ± 0.0033 mm, 0.0250 ± 0.0043 mm and 0.0250 ± 0.0017 mm respectively, which all had significant difference (P < 0.0001) compared with those in G2 model group, and the inhibitory rates were 82.65%, 74.49% and 74.49% respectively.

### 2) Histopathological scoring of ears of experimental animals

**Table 6 Histopathological scoring and quantification of ear thickness**

| Groups | | Hyperkeratosis of follicular infundibulum | Thickening of epidermis and inflammatory cell infiltration | Enlargement of follicular orifice | Total score |
|---|---|---|---|---|---|
| G1-normal group | Mean | 0.00**** | 0.00**** | 0.00**** | 0.00**** |
| | SEM | 0.00 | 0.00 | 0.00 | 0.00 |
| G2-model group | Mean | 1.90 | 2.10 | 2.00 | 6.00 |
| | SEM | 0.23 | 0.23 | 0.26 | 0.67 |
| G3-clobetasol propionate ointment group | Mean | 1.20* | 0.10**** | 0.80*** | 2.10**** |
| | SEM | 0.29 | 0.10 | 0.20 | 0.46 |
| G4-0.5% compound I group | Mean | 0.90** | 1.00**** | 0.90** | 2.80**** |
| | SEM | 0.18 | 0.21 | 0.18 | 0.55 |
| G5-1% compound I group | Mean | 1.20* | 1.30** | 1.50 | 4.00* |
| | SEM | 0.13 | 0.15 | 0.17 | 0.39 |
| G6-2% compound I group | Mean | 0.60**** | 1.10*** | 1.30 | 3.00*** |
| | SEM | 0.16 | 0.10 | 0.26 | 0.42 |

Results: as shown in Table 6, after the *in-vivo* experiment, the right ear tissues of all the experimental animals were collected, fixed with formaldehyde, embedded with paraffin, sectioned and stained with H&E, and then scored according to the pathological evaluation criteria (Table 4). The pathological diagnosis of the ear tissues showed that the ear tissues of the animals in G2 model group showed a dense keratinous substance of follicular infundibulum, thickening of superficial squamous epithelium, inflammatory cell infiltration and enlargement of follicular orifice, and each index for pathological scoring was obviously increased compared to that in G1 normal group (P < 0.0001).

In positive drug clobetasol propionate ointment group, the keratinous substance of follicular infundibulum was significantly reduced and the histopathological scoring was significantly different from that in G2 model group (1.20 ± 0.29 vs. 1.90 ± 0.23, P = 0.0476). The compound I topical preparation had a remarkable amelioration effect on the pathological indication of hyperkeratosis of follicular infundibulum of the ear tissues at concentrations of 0.5%, 1% and 2%, and the pathological scores were 0.90 ± 0.18 (P = 0.0021), 1.20 ± 0.13 (P = 0.0476) and 0.60 ± 0.16 (P < 0.0001) respectively. Each administration treatment group had a good therapeutic effect on the pathological phenomena of the thickening of superficial squamous epithelium and inflammatory cell infiltration caused by FITC modeling. The pathological scores of the indexes for the positive drug clobetasol propionate ointment group and 0.5%, 1% and 2% compound I groups were 0.10 ± 0.10, 1.00 ± 0.21, 1.30 ± 0.15 and 1.10 ± 0.10 respectively with significant differences (P < 0.0001, P < 0.0001, P = 0.0025 and P = 0.0001) compared to that in G2 model group (2.10 ± 0.23). The positive drug clobetasol propionate ointment group and 0.5% compound I group had a significant amelioration effect on the enlargement of follicular orifice with significant differences (P = 0.0004 and P = 0.0011) compared to G2 model group (2.00 ± 0.26), and the pathological scores were 0.80 ± 0.20 and 0.90 ± 0.18 respectively. The 1% and 2% compound I groups also had an amelioration effect on the enlargement of follicular orifice with the pathological scores of 1.50 ± 0.17 and 1.30 ± 0.26 respectively. For each administration treatment group, the total pathological score of the ear tissues of the experimental animals was significantly reduced. The total pathological scores of the positive drug clobetasol propionate ointment group and 0.5%, 1% and 2% compound I groups were 2.10 ± 0.46, 2.80 ± 0.55, 4.00 ± 0.39 and 3.00 ± 0.42 respectively with significant differences (P < 0.0001, P < 0.0001, P = 0.0156 and P = 0.0001) compared to that in G2 model group (6.00 ± 0.67).

In summary, the 0.5%, 1% and 2% compound I topical preparations had significant therapeutic effects on the development of the FITC-induced mouse atopic dermatitis.

### Example 2 Therapeutic effect of compound I gel on monobenzone-induced mouse vitiligo model

### 1. Objective

To evaluate the therapeutic effect of compound I gel on a monobenzone-induced mouse vitiligo model

### 2. Method

1) Animals: C57BL/6 mice, specific pathogen-free (SPF), with equal numbers of females and males. The mice were 6-8 weeks old at grouping, with 10 mice per group. The animals were provided by Charles River Laboratories.
2) Reagents, positive drugs and test drugs

### A. Reagents

The sources of the reagents are shown in Table 7 below.

**Table 7 Sources of reagents**

| Name | Supplier |
|---|---|
| 40% monobenzone depigmentation ointment | Xinzhuangyan |
| 0.9% sodium chloride injection | Guangxi Yuyuan Pharmaceutical |
| Xylene | Tianjin Damao Chemical Reagent Factory |
| Absolute ethanol | Xilong Scientific Co., Ltd. |
| 95% ethanol | Tianjin Damao Chemical Reagent Factory |
| Hematoxylin dye liquor | ZSGB-BIO |
| Scott bluing reagent | Solarbio |
| Eosin dye liquor | Solarbio |
| 4% paraformaldehyde | Biosharp |

B. Positive drug: tacrolimus ointment (0.03% tacrolimus), which was provided by LEO Laboratories Ltd.

C. Test drug: Compound I gel with the formulation that was the same as that in Table 2, which was provided by Guangzhou JOYO Pharmatech Co., Ltd.

### 3) Modeling method

Monobenzone induction method: after 1 week of adaptive feeding of all the animals, modeling was performed and all black hairs on the back of the mice were removed by a shaver. 10 animals were randomly selected as a normal control group and applied with 50 mg of vaseline once a day; and the remaining 80 animals were used for modeling and applied with 50 mg of 40% monobenzone depigmentation ointment once a day. The applying area of the normal control group and the model group was 2 cm*3 cm of the fixed part of the back, and the modeling was continuously performed for 103 days. In order to prevent the phenomenon that the animals lick the drugs, after the animals were applied with a modeling agent, they needed to be separately placed in an independent cage without padding for a period of time to naturally dry the modeling agent, and then they were placed in a rearing cage for normal rearing. During the modeling process, new hairs grown in the alopecic areas of the backs of the mice were shaved by the shaver and then applied with the drug.

### 4) Grouping and administration

The 40% monobenzone depigmentation ointment was applied every day. After modeling for 53 days, according to a depigmentation area score (see scoring criteria of Table 9), 70 animals successfully modeled in modeling group (score ≥ 2) were randomly divided into 7 groups with 10 mice per group. The mean depigmentation area score of the animals in each group was basically consistent before the administration, and the day of grouping was defined as day 0 of the administration treatment period. The details of the experimental grouping are shown in Table 8 below.

**Table 8 Grouping and administration regimen**

| Groups | Numbe r of animal s | Modeling^{a} | | Drug treatment^{a} | | | |
|---|---|---|---|---|---|---|---|
| | | | Name of drugs | Specificatio n | Administrati on dose (mg) | Drug conten t (mg) | Administrati on method |
| G1: Normal control group | 10 | Vaseline | - | - | - | - | - |
| G2: Model control group | 10 | Modeling by 40% monobenzo ne ointment | - | - | - | - | - |
| G3: 0.03% tacrolimu s treatment group | 10 | | Tacrolimu s ointment | 10 g:3 mg | 30 | 0.009 | Topically applied twice a day consecutivel y for 50 days (D1-D50) |
| G4: 1.5% ruxolitini b treatment group | 10 | | Ruxolitini b ointment | 15 g:225 mg | 30 | 0.45 | |
| G5: Blank preparatio n treatment group | 10 | | Blank gel | - | 30 | - | |
| G6: 0.5% compoun d I treatment group | 10 | | Compoun d I gel | 15 g:75 mg | 30 | 0.15 | |
| G7: 1% compoun d I | 10 | | Compoun d I gel | 15 g:150 mg | 30 | 0.3 | |
| treatment group | | | | | | | |
| G8: 2% compoun d I treatment group | 10 | | Compoun d I gel | 15 g:300 mg | 30 | 0.6 | |

| | | | | | | | |
|---|---|---|---|---|---|---|---|
| a: the applying sequence of the modeling agent and the treatment drug daily was as follows: the drug was applied once in the morning and evening every day at an interval of 6 hours or more. The modeling agent was applied once every afternoon. The applying position of the treatment drug was completely consistent with that of the modeling agent (2 cm*3 cm). | | | | | | | |

During the whole experiment process, the mice in normal control group (G1) were applied with 50 mg of vaseline once a day; and the mice in each model group (G2-G8) were applied with 50 mg of 40% monobenzone ointment once a day. After modeling for 53 days, the mice were grouped and subjected to drug treatment after grouping consecutively for 50 days. The modeling operation was maintained during the drug intervention period. The mice were applied with the drug twice a day for treatment at an interval of 6 hours or more. The drug treatment of each group was as follows:
(1) G1: normal control group: no normal saline washing and no drug administration;
(2) G2: model control group (i.e., vitiligo model normal saline group): the skin was washed with normal saline and dried, twice daily per mouse, without drug administration;
(3) G3: 0.03% tacrolimus treatment group (i.e., vitiligo model positive control drug group): the skin was applied with 0.03% tacrolimus ointment after being washed with normal saline and dried at 30 mg per dose, twice daily per mouse;
(4) G4: 1.5% ruxolitinib treatment group (i.e., vitiligo model reference compound ointment group): the skin was applied with reference drug ruxolitinib ointment after being washed with normal saline and dried at 30 mg per dose, twice daily per mouse;
(5) G5: blank preparation treatment group (i.e., vitiligo model blank gel group): the skin was applied with blank gel after being washed with normal saline and dried at 30 mg per dose, twice daily per mouse;
(6) G6: 0.5% compound I treatment group (i.e., vitiligo model low-dose compound gel group): the skin was applied with low-dose compound gel after being washed with normal saline and dried at 30 mg per dose, twice daily per mouse;
(7) G7: 1% compound I treatment group (i.e., vitiligo model medium-dose compound gel group): the skin was applied with medium-dose compound gel after being washed with normal saline and dried at 30 mg per dose, twice daily per mouse; and
(8) G8: 2% compound I treatment group (i.e., vitiligo model high-dose compound gel group): the skin was applied with high-dose compound gel after being washed with normal saline and dried at 30 mg per dose, twice daily per mouse; and 5) endpoint sample collection, histopathological analysis and hydroxyproline content analysis.

### 5) Depigmentation scoring of mice with vitiligo

Pictures were taken once each before administration and on day 10, 20, 30, 35, 38, 41, 44, 47 and 50 of the treatment, and were scored. Evaluation criteria of therapeutic effects were as follows: the part of the hair depigmentation area was divided into a treated part and an untreated part. The total score of each part was 5 and each mouse had a total score of 10.

**Table 9 Scoring criteria for hair depigmentation in mice with vitiligo**

| Score | Proportion of hair depigmentation area^{a} |
|---|---|
| 0 | No hair depigmentation |
| 1 | 0-10% of hair depigmentation area |
| 2 | 11 %-25% of hair depigmentation area |
| 3 | 26%-50% of hair depigmentation area |
| 4 | 51%-75% of hair depigmentation area |
| 5 | 76%-100% of hair depigmentation area |

| | |
|---|---|
| a: the evaluation method of the proportion of the depigmentation area of the modeling part was that the total area (6 cm²) of the modeling area was taken as a denominator. The percent of the hair depigmentation area of the modeling part = the depigmentation area in the modeling area/the total area of the modeling area* 100%. | |

### 6) Endpoint sample collection

The mice were euthanized within 2-4 hours after the last administration, and approximately 1 cm² of the skin in the center of the depilated part of the mice was taken and fixed with 4% paraformaldehyde.

### 7) Histopathological analysis

The animal skin tissues were fixed with 4% paraformaldehyde, dehydrated, embedded with paraffin, sectioned and then subjected to HE staining. The stained sections were photographed by white light panoramic scanning, histopathological changes of the skin tissues and inflammatory cell infiltration were observed, and the number of melanin-containing hair follicles and/or the proportion of the melanin-containing hair follicles in the skin were quantitatively analyzed.

The panoramic scanning files were opened by using CaseViewer software, a picture view with the length of about 3.8 mm was randomly selected, and the pictures were saved. The number of all the hair follicles and the number of the melanin-containing hair follicles in the picture were counted respectively by using ipwin32 software. The proportion of the melanin-containing hair follicles was calculated according to the following formula:

Proportion of melanin-containing hair follicles = number of melanin-containing follicles/number of all follicles

### 3. Results

### 1) Depigmentation area scoring of mice with vitiligo

**Table 10 Mean values of hair depigmentation area scoring of mice during therapeutic administration period of compound I topical preparation**

| Groups | | Day 0 | Day 10 | Day 20 | Day 30 | Day 35 | Day 38 | Day 41 | Day 44 | Day 47 | Day 50 |
|---|---|---|---|---|---|---|---|---|---|---|---|
| Normal control group | Mean | 0.00 | 0.00 | 0.00 | 0.00 | 0.00 | 0.00 | 0.00 | 0.00 | 0.00 | 0.00 |
| | SEM | 0.00 | 0.00 | 0.00 | 0.00 | 0.00 | 0.00 | 0.00 | 0.00 | 0.00 | 0.00 |
| Model control group | Mean | 4.00 ## | 5.00 ## | 5.50 ## | 5.50 ## | 5.30 ## | 5.50 ## | 4.70 ## | 4.80 ## | 5.00 ## | 5.10 ## |
| | SEM | 0.37 | 0.37 | 0.17 | 0.17 | 0.21 | 0.17 | 0.15 | 0.20 | 0.15 | 0.18 |
| 0.03% | Mean | 3.60 | 3.50 | 3.40 | 3.40 | 2.60 | 2.50 | 2.30 | 2.20 | 2.20 | 2.20 |
| tacrolimus treatment group | | | ** | ** | ** | ** | ** | ** | ** | ** | ** |
| | SEM | 0.31 | 0.27 | 0.16 | 0.22 | 0.16 | 0.17 | 0.15 | 0.13 | 0.13 | 0.13 |
| 1.5% ruxolitinib treatment group | Mean | 3.90 | 3.80 ** | 3.70 ** | 3.40 ** | 2.90 ** | 2.60 ** | 2.40 ** | 2.30 ** | 2.20 ** | 2.20 ** |
| | SEM | 0.41 | 0.20 | 0.26 | 0.22 | 0.18 | 0.16 | 0.16 | 0.15 | 0.13 | 0.13 |
| Blank preparation treatment group | Mean | 3.90 ** | 4.60 | 4.00 ** | 4.80 * | 4.60 * | 4.90 * | 4.30 | 4.40 | 4.30 ** | 4.30 ** |
| | SEM | 0.28 | 0.27 | 0.26 | 0.25 | 0.27 | 0.28 | 0.21 | 0.16 | 0.15 | 0.15 |
| 0.5% compound I treatment group | Mean | 3.80 | 3.60 ** | 3.60 ** | 3.30 ** | 3.20 ** | 3.20 ** | 2.90 ** | 2.80 ** | 2.60 ** | 2.50 ** |
| | SEM | 0.33 | 0.22 | 0.27 | 0.26 | 0.29 | 0.29 | 0.23 | 0.20 | 0.16 | 0.17 |
| 1% compound I treatment group | Mean | 3.70 | 3.90 ** | 3.60 ** | 3.40 ** | 3.10 ** | 2.90 ** | 2.60 ** | 2.50 ** | 2.40 ** | 2.30 ** |
| | SEM | 0.33 | 0.31 | 0.31 | 0.16 | 0.18 | 0.18 | 0.22 | 0.17 | 0.16 | 0.15 |
| 2% compound I treatment group | Mean | 3.80 | 4.00 ** | 3.50 ** | 3.10 ** | 2.90 ** | 2.60 ** | 2.30 ** | 2.20 ** | 2.20 ** | 2.10 ** |
| | SEM | 0.33 | 0.26 | 0.17 | 0.28 | 0.31 | 0.27 | 0.15 | 0.13 | 0.13 | 0.10 |

| | | | | | | | | | | | |
|---|---|---|---|---|---|---|---|---|---|---|---|
| Note: #, compared to the normal control group (#, P < 0.05; ##, P < 0.01); *, compared to the model control group (*, P < 0.05; **, P < 0.01) | | | | | | | | | | | |

Results: after the experimental animals were modeled by the monobenzone, spot-shaped depigmentation gradually appeared at the modeling part, depigmentation also appeared at non-modeling parts such as ears and tails, and the depigmentation did not appear in the normal control group. After successful modeling, the depigmentation area scoring was performed on day 0 (the day of grouping and before therapeutic administration), day 10, day 20, day 30, day 35, day 38, day 41, day 44, day 47 and day 50 respectively. As shown in Table 10, the mean value of the depigmentation scores was 4.0 ± 0.37 on day 0, namely on the day of grouping the animals in the model control group, and reached a platform of 5.5 ± 0.17 after administration on day 20, which proved that the mouse vitiligo model was successfully constructed.

The therapeutic effect was assessed by comparing the area under the curve (AUC) of the hair depigmentation area score for each group of the mice with the total score of the hair depigmentation area of the administration on day 50. As shown in Table 10, compared with the model control group, the positive drug 0.03% tacrolimus group had a significant amelioration effect on the development of diseases, the depigmentation area scores showed significantly differences from day 10 to day 50, and the reduction rate of the depigmentation area score was 56.9% on day 50 of drug administration. For the reference drug 1.5% ruxolitinib group, the drug was topically administered for treatment at a dose of 30 mg (5 mg/cm²), the depigmentation area scores showed significantly differences from that of the blank preparation group from day 10 to day 50, and the mean value of the reduction rate of the depigmentation area score was 56.9% (P < 0.01) on day 50 of administration. In each compound I treatment groups, the drug was topically administered for treatment at a dose of 30 mg (5 mg/cm²). Compared with the model control group, the 0.5%, 1%, and 2% Compound I groups significantly reduced the depigmentation area scores in vitiligo mice from Day 10 to Day 50. On Day 50 of treatment, the mean inhibitory rates of depigmentation area scores were 52.0% (P<0.01), 54.9% (P<0.01) and 58.8% (P < 0.01), respectively. Compared with the blank preparation group, the mean inhibitory rates of the depigmentation area scores of the compound I topical preparation low-, medium- and high-concentration groups were 41.9% (P < 0.01), 46.5% (P < 0.01) and 51.2% (P < 0.01) on day 50 of administration, respectively. Therefore, the Compound I topical preparation can obviously ameliorate the clinical symptoms of the mouse vitiligo model.

### 2) Skin pathological analysis

**Table 11 Number of melanin-containing hair follicles and proportion of melanin-containing hair follicles after therapeutic administration of Compound I topical preparation**

| Groups | | Number of melanin-containing hair follicles | Proportion of melanin-containing hair follicles (%) |
|---|---|---|---|
| Normal control group | Mean | 58.3 | 46.4 |
| | SEM | 23.3 | 8.8 |
| Model control group | Mean | 6.7# | 14.1 # |
| | SEM | 3.3 | 6.7 |
| 0.03% tacrolimus treatment group | Mean | 4.3 | 7.0 |
| | SEM | 3.3 | 4.00 |
| 1.5% ruxolitinib treatment group | Mean | 41.7 | 29.8 |
| | SEM | 16.7 | 9.9 |
| Blank preparation treatment group | Mean | 19.5 | 20.7 |
| | SEM | 9.5 | 6.0 |
| 1% compound I treatment group | Mean | 30.3 | 29.7 |
| | SEM | 14.8 | 9.1 |
| 2% compound I treatment group | Mean | 26.9* | 30.6 |
| | SEM | 7.4 | 6.2 |

| | | | |
|---|---|---|---|
| Notes: #, compared to the normal control group (#, P < 0.05; ##, P < 0.01); *, P < 0.05 compared to the model control group. | | | |

### Results:

The skins of the mice in the modeling area were collected at a test endpoint. Pathological observation was performed on the skin through HE staining, and the number of the melanin-containing hair follicles and the proportion of the melanin-containing hair follicles were subjected to quantitative analysis. As shown in Table 11, compared to the normal control group, the mean value of the number of the melanin-containing hair follicles in the model control group was obviously reduced (6.7 ± 3.3 vs. 58.3 ± 23.3, P < 0.05), and the mean value of the proportion of the melanin-containing hair follicles was obviously reduced (14.1 ± 6.7% vs. 46.4 ± 8.8%, P < 0.05), which proved the successful construction of the mouse vitiligo model.

The statistical results of the number of the melanin-containing hair follicles showed that compared to the model control group, the mean value of the number of the melanin-containing hair follicles was not obviously changed in the 0.03% tacrolimus group, was increased to 41.7±16.7 in the reference drug 1.5% ruxolitinib group which was about 6 times that of the model control group (P = 0.142), and was close to the mean value level of the normal control group. The numbers of the melanin-containing hair follicles of the blank preparation group, and the 1% and 2% Compound I gel groups were 19.5 ± 9.5, 30.3 ± 14.8 and 26.9 ± 7.4 respectively. The number of the melanin-containing hair follicles in the 2% compound I group was about 4 times (P = 0.025) that of the model control group, which was increased by 37.9% compared to the blank preparation group.

The statistical results of the proportion of the melanin-containing hair follicles showed that compared to the model control, the mean value of the proportion of the melanin-containing hair follicles in the 0.03% tacrolimus group did not change obviously. The proportion of the melanin-containing hair follicles of the reference drug 1.5% ruxolitinib group was increased to 29.8 ± 9.9% which was about twice that of the model control group (P = 0.142) and was close to the mean value level of the normal control group. The proportions of the melanin-containing hair follicles of the blank preparation group, and 1% and 2% compound I groups were 20.7 ± 6.0%, 29.7 ± 9.1% and 30.6 ± 6.2% respectively. The proportion of the melanin-containing hair follicles of the 2% compound I group was about twice that of the model control group (P = 0.142), indicating that the 2% compound I had the therapeutic effect equivalent to that of the ruxolitinib ointment.

According to the skin pathological observation and quantitative analysis of the number of the melanin-containing hair follicles and the proportion of the melanin-containing hair follicles, 1% and 2% compounds I can ameliorate the reduction of the melanin-containing hair follicles in vitiligo model, indicating that the compound I has the therapeutic effect that is equivalent to that of 1.5% ruxolitinib and superior to that of 0.03% tacrolimus.

### Example 3 In-vivo pharmacodynamic study of compound on alopecia areata mouse model

### 1. Objective

To evaluate the therapeutic effect of test compound I gel on an imiquimod-induced C3H/HeJ mice alopecia areata model

### 2. Method

1) Animals: female C3H/He mice, SPF, aged 6-8 weeks at grouping. The animals were provided by Beijing Viewsolid Biotech.
2) Reagents, positive drugs and test drugs

### A. Reagents

The sources of the reagents are shown in Table 12 below.

**Table 12 Sources of reagents**

| Name | Item No. | Manufacturer |
|---|---|---|
| HE dye liquor | KGA224 | Jiangsu Keygen Biotech |
| Xylene | 10023418 | Sinopharm Chemical Reagent Co., Ltd. |
| Absolute ethanol | 10009218 | Beijing Solarbio Science&Technology Co., Ltd. |
| Normal saline | IN9000 | Beijing Solarbio Science&Technology Co., Ltd. |

B. Positive drug: Baricitinib, which was provided by BioChemPartner.

C. Test drug: Compound I, which was provided by Guangzhou JOYO Pharmatech Co., Ltd.

### 3) Modeling method

After one week of adaptive feeding of C3H/HeJ mice: modeling was performed by applying imiquimod ointment to the neck, 3 times a week. The mice were carefully taken out, and about 0.05 g of 5% imiquimod ointment was dipped with a clean medical cotton swab and then applied evenly to the skin on the neck over a range of about 1.5 cm×1.5 cm. Before each application, the local part was cleaned by normal saline to remove the residual drug in the previous time. The continuous administration time was no longer than 10 weeks. When alopecia areata with an area size > 1 cm×1 cm was formed at the administration part, the administration was stopped, proving that a preparation model was successful. The mice in the normal control group were modeled by using blank ointment (vaseline ointment).

### 4) Grouping and administration

The grouping and administration are shown in Table 13 below.

**Table 13 Grouping and administration**

| Groups | Number of animals | Drug | Administration route | Administration dose | Concentration (mg/mL) | Administration frequency and cycle |
|---|---|---|---|---|---|---|
| Model control group | 5 | Test sample solvent^{a} | Oral administration by gavage | - | - | After successful modeling, administered twice a day consecutively for one month |
| Positive control drug treatment group | 5 | Baricitinib^{b} | Oral administration by gavage | 10 mg/kg | 1.0 | |
| Test sample high-dose treatment group | 5 | Compound I^{b} | Oral administration by gavage | 10 mg/kg | 1.0 | |
| Test sample low-dose treatment group | 3 | Compound I^{b} | Oral administration by gavage | 3 mg/kg | 0.3 | |

| | | | | | | |
|---|---|---|---|---|---|---|
| Note: a: a test sample solvent was an aqueous solution of 0.5% (w/v, the percentage of the mass of hydroxypropyl methylcellulose accounting for the volume of the solution) of hydroxypropyl methylcellulose, 0.5% (w/v, the percentage of the mass of polyvinylpyrrolidone accounting for the volume of the solution) of polyvinylpyrrolidone and 0.2% (w/v, the percentage of the mass of lauryl sodium sulfate accounting for the volume of the solution) of lauryl sodium sulfate; and b: the required concentration was prepared by using an aqueous solution of 0.5% (w/v) hydroxypropyl methylcellulose, 0.5% (w/v) polyvinylpyrrolidone and 0.2% (w/v) sodium dodecyl sulfate was used as a solvent to prepare to the required concentration. | | | | | | |

### 5) Clinical indication determination

### A. Gross photograph

During the period from the first administration to the end of the last administration after the alopecia areata modeling was successful, the alopecia areata part of each mouse was photographed once every 3 days, the photographing distance, angle and magnification times were fixed, and the hair growth of the alopecic areas on the backs of the mice was recorded.

### B. Evaluation of newly grown hairs

Visual quantification of the alopecia area images was performed using ImageJ software. The hair growth area (a) and hair loss area (b) were calculated. The hair growth rate was counted and analyzed: hair growth rate (%) = (a/b)* 100%. The newly grown hairs at the alopecia areata part of the mice in each group were scored based on the hair growth rate. Scoring criteria are as shown in Table 14 below.

**Table 14 Scoring criteria of newly grown hairs**

| Scoring criteria of newly grown hairs | |
|---|---|
| Score | Hair growth |
| 0 | No growth observed |
| 1 | The hair growth rate reaches 0-20% |
| 2 | The hair growth rate reaches 20-40% |
| 3 | The hair growth rate reaches 40-60% |
| 4 | The hair growth rate reaches 60-80% |
| 5 | The hair growth rate reaches 80-100% |

### 6) Endpoint sample collection and histopathological analysis

### A. Endpoint sample collection

After the animals were anesthetized and euthanized, the complete skin tissue of the alopecic area was cut along the junction between the normal skin of the back of the mouse and the alopecic area and perpendicular to the skin surface. A part of the tissue was taken, conventionally dehydrated and embedded. The remaining tissue was stored in a refrigerator at -80°C for later use.

### B. Histopathological analysis

The embedded skin tissue was cut into 3 µm-thick sections, and then the sections were dewaxed in xylene, hydrated by gradient ethanol, H&E stained, dehydrated, cleared and mounted. The hair follicles in skin tissue sections from each group were microscopically observed and photographed. The mean number of the hair follicles was calculated using Image J software.

### 4. Results

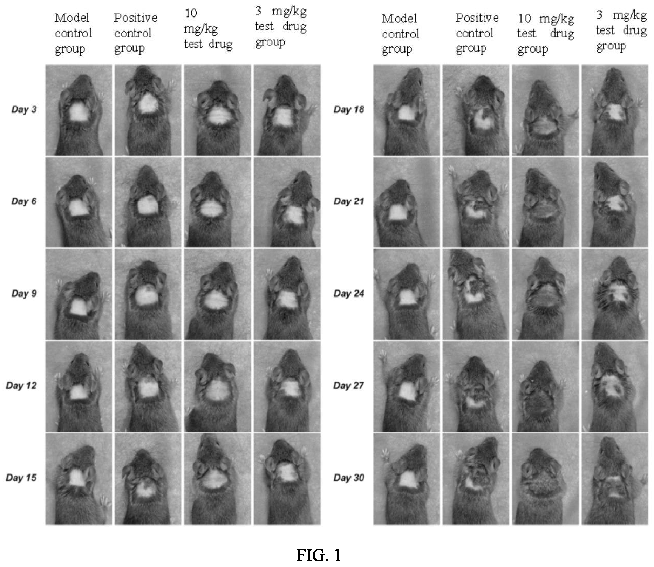

1) The hair growth of the alopecia areata part is seen in FIG. 1, and the effect of the test drug on the hair growth of the mice is shown in Table 15.

**Table 15 Effect of test drug on hair growth of mice**

| Grouping | Hair growth rate (%) | Newly grown hair score |
|---|---|---|
| Model control group | 6.45 + 1.2 | 1 + 0 |
| Positive control group | 69.58 + 3.23**** | 4.2 + 0.2**** |
| 10 mg/kg test drug group | 83.93 + 5.58**** | 4.6 ± 0.24**** |
| 3 mg/kg test drug group | 71.62 + 5.56**** | 4 + 0**** |

| | | |
|---|---|---|
| Note: the results were expressed as Mean ± SEM; compared to the model control group, **** P < 0.0001. | | |

### Results:

After 8 weeks of modeling with the imiquimod ointment (3 times a week), the neck backs of all the mice showed distinct and well-defined patchy hair loss, indicating the successful alopecia areata modeling. After the mice were randomly grouped, the administration treatment was started consecutively for 1 month, the day of the administration was defined as Day 0, and the alopecia areata part was photographed every 3 days to record the hair growth of the mice. By the endpoint of the experiment (Day 30), the positive drug and the test compound can obviously ameliorate the alopecia areata disease of the mice under the set treatment scheme, the hair growth at the alopecic area was obvious, and the amelioration effect of the test drug at the dose of 3 mg/kg was equivalent to that of the positive control drug at the dose of 10 mg/kg (FIG. 1); compared to the model control group, the hair growth rates of the alopecia areata parts of the positive drug, the 10 mg/kg test drug and the 3 mg/kg test drug groups (69.58 ± 3.23 vs 6.45 ± 1.2, P < 0.0001; 83.93 ± 5.58 vs 6.45 ± 1.2, P < 0.0001 and 71.62 ± 5.56 vs 6.45 ± 1.2, P < 0.0001) and the newly grown hair scores (4.2 ± 0.2 vs 1 ± 0, P < 0.0001; 4.6 ± 0.24 vs 1±0, P < 0.0001 and 4 ± 0 vs 1 ± 0, P < 0.0001) were all significantly improved (Table 15).

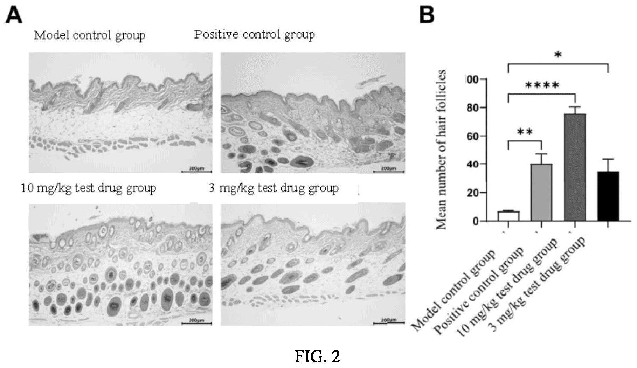

2) The pathological analysis of the skin at the alopecia areata part is seen in FIG. 2, and the effect of the test drug on the number of the hair follicles in the skin at the alopecia areata part of the mice is shown in Table 16.

**Table 16 Effect of test drug on number of hair follicles in skin at alopecia areata part of mice**

| Grouping | Mean number of hair follicles |
|---|---|
| Model control group | 7.2 ± 0.37 |
| Positive control group | 40 ± 7.36** |
| 10 mg/kg test drug group | 76 ± 4.39**** |
| 3 mg/kg test drug group | 35 ± 8.89* |

| | |
|---|---|
| Note: the results were expressed as Mean ± SEM; compared to the model control, * P < 0.05, ** P < 0.01 and **** P < 0.0001. | |

Results: the mice in each group were euthanized within 4 hours after the last administration (day 30) was ended, the skin tissues of the alopecia areata modeling part were collected and conventionally fixed, the pathological changes of the skin tissues of the alopecia areata part were observed, the mean number of the hair follicles was calculated using Image J software, and then the differences were recorded and counted. The results showed that there was no obvious inflammatory reaction in the skin tissues of the alopecia areata part in each experimental group. Compared to the model control group, the mean numbers of the hair follicles of the skins of the alopecia areata parts in the positive drug, the 10 mg/kg test drug and the 3 mg/kg test drug groups (40 ± 7.36 vs 7.2 ± 0.37, P < 0.01; 76 ± 4.39 vs 7.2 ± 0.37, P < 0.0001 and 35 ± 8.89 vs 7.2±0.37, P < 0.05) were significantly increased.

The experimental results showed that the test compound had obvious therapeutic effect on the mouse alopecia areata model, and the therapeutic effect was superior to that of the positive drug baricitinib.

### Example 4 Phase II clinical study on effectiveness and safety of compound shown in Formula I in adult patients with moderate to severe atopic dermatitis

Primary objective: to evaluate the effectiveness of an oral compound tablet shown in Formula I in adult patients with moderate to severe atopic dermatitis. Secondary objectives: (1) to evaluate the safety of the oral compound tablet shown in Formula I in adult patients with moderate to severe atopic dermatitis; and (2) to evaluate the pharmacokinetics (PK) of the oral compound tablet shown in Formula I in adult patients with moderate to severe atopic dermatitis. Exploratory objective: to explore the biomarker changes of the oral compound tablet shown in Formula I in adult patients with moderate to severe atopic dermatitis.

Primary endpoint: at week 12, the proportion of subjects with an eczema area and severity index (EASI) was improved by at least 75% (EASI 75) compared with a baseline.

Secondary endpoints: (1) effectiveness endpoint: the proportion of subjects with EASI75 at each time point, the proportion of subjects with IGA responses at each time point and the proportion of subjects with EASI were improved by at least 50% (EASI50)/90% (EASI90) compared with the baseline at each time point, the proportion of subjects with Worst Itch/Pruritus Numeric Rating Scale (WI-NRS/WP-NRS) was improved by ≥ 4 points compared with the baseline at each time point, the changes of EASI/total area of skin lesion/WI-NRS/Dermatology Life Quality Index (DLQI)/Patient Oriented Eczema Measure (POEM) compared with the baseline at each time point, onset time of WI-NRS improved by ≥ 4 points compared with the baseline, the proportion of subjects in need of remedial treatment during study treatment; (2) safety endpoints: a, treatment emergent adverse event (TEAE), treatment-related adverse event (TRAE), severe adverse event (SAE), adverse event of special interest (AESI) and incidence of adverse events leading to termination of study; and b, the incidence of clinically significant abnormalities and changes compared with the baseline in examination results such as vital signs, laboratories and electrocardiograms; and (3) pharmacokinetic endpoint: the PK of the compound shown in Formula I in adult subjects with moderate to severe atopic dermatitis.

Exploratory endpoint: the changes in the pharmacodynamic and disease-associated biomarkers [blood TARC (CCL17) and the like] at each time point.

Study population: target populations for this study are adult subjects with moderate to severe atopic dermatitis.

### Inclusion criteria:

1. The subjects are aged 18 to 75 years inclusive, male or female.
2. the body weights of the subjects are ≥ 40 kg;
3. During the screening, the subjects meet the Williams standard of atopic dermatitis, that is, the atopic dermatitis is developed for at least 1 year, and the severity degree is moderate or severe; and the definition of moderate to severe atopic dermatitis: all of the following four criteria must be met: Investigator's Global Assessment (IGA) score ≥ 3, Eczema Area and Severity Index (EASI) score ≥ 16, average peak pruritus NRS score ≥ 4 over the past week, and total affected body surface area ≥ 10% BSA (BSA: body surface area; a mean human body surface area of 1.6 m² was used as the reference in this trial); and
4. Within at least 6 months prior to the screening, a well-defined medical history shows a topical drug (including corticosteroids, calcineurin inhibitors and the like) is regularly used for at least 4 weeks (or the maximum recommended course of treatment in the product prescription information), which still fails to achieve or maintain remission or low disease activity (IGA0-2 scores), or systemic treatment to control the disease is still required, or topical treatment is not medically advised.

After the screening, the qualified subjects were randomly assigned to a 30 mg compound tablet group shown in Formula I (30 mg taken once a day), a 15 mg compound tablet group shown in Formula I (15 mg taken once a day), a 10 mg compound tablet group shown in Formula I (10 mg taken once a day) or a placebo group. The subjects in each group were administered within 30 minutes from breakfast each day for 12 weeks.

## Claims

1. Use of a compound shown in Formula I, a pharmaceutically acceptable salt thereof or a pharmaceutical composition in the manufacture of a drug for treating and/or preventing an autoimmune skin disease, wherein the pharmaceutical composition comprises the compound shown in Formula I or the pharmaceutically acceptable salt thereof and at least one pharmaceutically acceptable carrier, and the autoimmune skin disease is one or more of atopic dermatitis, vitiligo and alopecia areata,

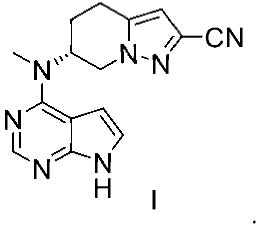

2. The use according to claim 1, wherein the use satisfies one or more of the following conditions:
(1) the pharmaceutically acceptable salt is fumarate, adipate, phosphate, tartrate, maleate, hydrochloride, citrate, sulfate, methanesulfonate, benzenesulfonate or p-toluenesulfonate; and
(2) the pharmaceutical composition is an oral preparation or a topical preparation.

3. The use according to claim 1 or 2, wherein the use satisfies one or more of the following conditions:
(1) the pharmaceutically acceptable salt is sulfate;
(2) the pharmaceutical composition is a capsule, a tablet, a granule, a powder, a lotion, a tincture, a liniment, a paint, an ointment, a gel, a paste, a transdermal patch or an aerosol, preferably an ointment or a gel;
(3) the atopic dermatitis has one or more of the following characteristics:
(I) hyperkeratosis of follicular infundibulum;
(II) thickening of epidermis;
(III) inflammatory cell infiltration; and
(IV) enlargement of follicular orifice;
(4) the vitiligo is segmental vitiligo, non-segmental vitiligo, mixed vitiligo or indeterminate vitiligo;
(5) the alopecia areata is of a patch type, a net type, an ophiasis type, a central type, a reverse-ophiasis type, a diffuse type, alopecia totalis or alopecia universalis; and
(6) the pharmaceutical composition consists of the compound shown in Formula I, the pharmaceutically acceptable salt thereof and at least one pharmaceutically acceptable carrier.

4. The use according to any one of claims 1 to 3, wherein the use satisfies one or more of the following conditions:
(1) calculated as the compound shown in Formula I, each minimum drug content specification contains 0.1-200 mg of the compound shown in Formula I, such as 0.2 mg, 0.5 mg, 1 mg, 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg; preferably 1 mg, 5 mg, 10 mg, 15 mg, 30 mg, 45 mg, 60 mg, 75 mg, 90 mg or 120 mg; more preferably 5 mg, 10 mg, 15 mg, 30 mg, 45 mg, 60 mg, 75 mg or 90 mg; or calculated as the compound shown in Formula I, each minimum drug content specification contains 0.5 wt%-5.0 wt% of the compound shown in Formula I, such as 0.5 wt%-2 wt%;
(2) the administration frequency of the compound shown in Formula I, the pharmaceutically acceptable salt thereof or the pharmaceutical composition is once every two days, once every day, twice every day or three times every day, preferably once every day or twice every day; and
(3) the administration mode of the compound shown in Formula I, the pharmaceutically acceptable salt thereof or the pharmaceutical composition is oral administration, parenteral administration or transdermal administration, preferably oral administration or transdermal administration.

5. The use according to any one of claims 1 to 3, wherein the use satisfies one or more of the following conditions:
(1) when the compound shown in Formula I, the pharmaceutically acceptable salt thereof or the pharmaceutical composition is applied topically, the dose calculated as the compound shown in Formula I is 0.001-1 mg/cm², preferably 0.005-0.5 mg/cm², more preferably 0.01-0.2 mg/cm², such as 0.01 mg/cm², 0.03 mg/cm², 0.05 mg/cm², 0.07 mg/cm², 0.08 mg/cm², 0.1 mg/cm², 0.15 mg/cm² or 0.2 mg/cm²;
(2) when the compound shown in Formula I, the pharmaceutically acceptable salt thereof or the pharmaceutical composition is orally taken, the total daily dose calculated as the compound shown in Formula I is 5-200 mg, such as 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg; and
(3) the compound shown in Formula I or the pharmaceutically acceptable salt thereof is administered at 5-90 mg per dose, such as 5 mg, 10 mg, 15 mg, 30 mg, 45 mg, 60 mg, 75 mg, or 90 mg.

6. The use according to any one of claims 1 to 3, wherein the use satisfies one or more of the following conditions:
(1) when the autoimmune skin disease is atopic dermatitis, the dose calculated as the compound shown in Formula I is 0.005-0.5 mg/cm², preferably 0.01-0.2 mg/cm², such as 0.01 mg/cm², 0.03 mg/cm², 0.05 mg/cm², 0.07 mg/cm², 0.08 mg/cm², 0.1 mg/cm², 0.15 mg/cm² or 0.2 mg/cm²;
(2) when the autoimmune skin disease is vitiligo, the dose calculated as the compound shown in Formula I is 0.005-0.5 mg/cm², preferably 0.01-0.2 mg/cm², such as 0.01 mg/cm², 0.03 mg/cm², 0.05 mg/cm², 0.07 mg/cm², 0.08 mg/cm², 0.1 mg/cm², 0.15 mg/cm² or 0.2 mg/cm²;
(3) when the autoimmune skin disease is alopecia areata, the dose calculated as the compound shown in Formula I is 5-200 mg, such as 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg;
(4) when the autoimmune skin disease is atopic dermatitis or vitiligo, the administration mode of the compound shown in Formula I, the pharmaceutically acceptable salt thereof or the pharmaceutical composition is transdermal administration; and
(5) when the autoimmune skin disease is alopecia areata, the administration mode of the compound shown in Formula I, the pharmaceutically acceptable salt thereof or the pharmaceutical composition is oral administration, parenteral administration or transdermal administration, preferably oral administration.

7. The use according to any one of claims 1 to 3, wherein the use satisfies one or more of the following conditions:
(1) when the autoimmune skin disease is the atopic dermatitis, the compound shown in Formula I, the pharmaceutically acceptable salt thereof or the pharmaceutical composition is used by transdermal administration twice every day at the dose of 0.005-0.1 mg/cm² each time calculated as the compound shown in Formula I;
(2) when the autoimmune skin disease is vitiligo, the compound shown in Formula I, the pharmaceutically acceptable salt thereof or the pharmaceutical composition is used by transdermal administration twice every day at the dose of 0.005-0.1 mg/cm² each time calculated as the compound shown in Formula I; and
(3) when the autoimmune skin disease is alopecia areata, the compound shown in Formula I, the pharmaceutically acceptable salt thereof or the pharmaceutical composition is used by oral administration twice every day at the dose of 5-90 mg each time calculated as the compound shown in Formula I.

8. The use according to any one of claims 1 to 3, wherein the use satisfies one or two of the following conditions:
(1) when the pharmaceutical composition is the oral preparation, the dose of the compound shown in Formula I and the pharmaceutically acceptable salt thereof in the pharmaceutical composition is 5-200 mg calculated as the compound shown in Formula I; and
(2) when the pharmaceutical composition is the gel, the pharmaceutical composition is a pharmaceutical composition A comprising the following components:
an active ingredient, wherein the active ingredient is the compound shown in Formula I or the pharmaceutically acceptable salt thereof; and the active ingredient accounts for 0.5 wt%-2 wt% of the pharmaceutical composition calculated as the compound of formula I;
5.0 wt%-7.0 wt% of Sepineo P 600;
20.0 wt% of diethylene glycol monoethyl ether;
15.0 wt% of glycerol; and
propylene glycol q.s. to 100% of the total composition.

9. The use according to claim 8, wherein the pharmaceutical composition satisfies one or two of the following conditions:
(1) when the pharmaceutical composition is the oral preparation, the dose of the compound shown in Formula I and the pharmaceutically acceptable salt thereof in the pharmaceutical composition is 5 mg, 10 mg, 15 mg, 30 mg, 45 mg, 60 mg, 75 mg, 90 mg, 100 mg, 115 mg, 120 mg, 130 mg, 145 mg, 160 mg, 175 mg or 190 mg calculated as the compound shown in Formula I; and
(2) the pharmaceutical composition A is selected from any one of the followings:
the pharmaceutical composition A contains an active ingredient, wherein the active ingredient is the sulfate of the compound shown in Formula I and the active ingredient accounts for 2 wt% of the pharmaceutical composition calculated as the compound of formula I;
7.0 wt% of Sepineo P 600;
20.0 wt% of diethylene glycol monoethyl ether;
15.0 wt% of glycerol; and propylene glycol q.s. to 100% of the total composition;
or the pharmaceutical composition A contains an active ingredient, wherein the active ingredient is the sulfate of the compound shown in Formula I and the active ingredient accounts for 1 wt% of the pharmaceutical composition calculated as the compound of formula I;
6.0 wt% of Sepineo P 600;
20.0 wt% of diethylene glycol monoethyl ether;
15.0 wt% of glycerol; and propylene glycol q.s. to 100% of the total composition;
or the pharmaceutical composition A contains an active ingredient, wherein the active ingredient is the sulfate of the compound shown in Formula I and the active ingredient accounts for 0.5 wt% of the pharmaceutical composition calculated as the compound of formula I;
5.0 wt% of Sepineo P 600;
20.0 wt% of diethylene glycol monoethyl ether;
15.0 wt% of glycerol; and propylene glycol q.s. to 100% of the total composition.

10. A pharmaceutical composition as shown in the pharmaceutical composition A in the use according to claim 8 or 9.
